# EUROPEAN PATENT APPLICATION

(11) **EP 1 452 161 A1**
(43) Date of publication of application: **01.09.2004**
(21) Application number: 02762871.8
(22) Date of filing: 27.08.2002
(51) Int. Cl.: A61K 7/00, A61K 7/48, A61K 7/40, A61P 17/00, C08F 220/12, C08F 290/06

(54) **EXTERNAL PREPARATION FOR THE SKIN AND COPOLYMER TO BE USED THEREIN**

(30) Priority: 05.12.2001 JP 2001371324
(71) Applicant: POLA CHEMICAL INDUSTRIES, INC., Shizuoka-shi, Shizuoka 420-0914 (JP)
(72) Inventor: IYANAGI, Hirokazu, Pola Chemical Industries Inc., Yokohama-shi, anagawa 244-0812 (JP)
(74) Representative: Bannerman, David Gardner
(86) International application number: PCT/JP2002/008617
(87) International publication number: WO 2003/047535

(57) **Abstract**

An object of the present invention is to provide an external preparation for the skin, which shows an excellent effect of sufficiently protecting the living body against irritants that exist in the external environments, and a polymer material to be preferably used therein.

Provided is an external preparation for the skin obtained mixing a copolymer which comprises constituent units derived from monomers of the general formula (I), (II) and, if necessary, (IV) with the content of units (I) ranging from 5 to 25 wt % based on the total amount of all the constituent units, preferably with the contents of units (I), (II) and (IV) being 5 to 9.8 wt %, 30.2 to 45 wt %, and 50 to 60 wt %respectively, with other optional components. In the formula, R¹ is H or C1-3 alkyl; R² is C5-12 alkyl having 8 to 21 fluorine atoms; R³ is H or C1-3 alkyl; R⁴ is optionally hydroxylated C2-4 alkylene; R⁵ is H, a C1-20 aromatic group, a C1-20 aliphatic hydrocarbon group, or C1-20 acyl; and n is 4 to 50.

## Description

### Technical Field

The present invention relates to a copolymer and an external preparation for the skin which contains the same, and particularly relates to a copolymer useful in an external preparation for the skin to protect against irritation and an external preparation for the skin which contains the copolymer.

### Background Art

In recent years, external environments that surround us have changed and the chances to be exposed to chemical substances have remarkably increased in the living environment. Those chemical substances have had various undesirable influences on the living bodies. The bad influences on the living bodies include allergies and house-sick syndromes caused by the chemical substances. Reflecting the above-mentioned situations, those maladies are increasing more and more in recent years. Besides, in many cases, even in the case of a normal person, the protective function of the skin is decreased, that is, the skin is being sensitive, so the normal person is frequently troubled by considerable irritation with various chemical substances.

However, with respect to the above maladies, for example, it is actual to deal with the mucous membrane by protection with a mask or the like to prevent exposure while sacrificing the convenience of life, or with already-happened symptoms by symptomatic treatment, so that effective preventive measures have not yet taken. In addition, there is no effective method for sufficient and satisfactory protection of the sensitive skin from irritation.

In the field of external preparations for the skin, such as cosmetics, it has been known that those containing water-soluble polymers such as poly methacryloyl oxyethoxyphosphatidyl choline have protective effects on irritation by reinforcing the barrier function of the skin, but the protective effects achieved by them have not been brought out sufficiently.

### Disclosure of the Invention

The present invention has been made under such circumstances and an object of the present invention is to provide an external preparation for the skin, which shows an excellent effect of sufficiently protecting the living body against irritants that exist in the external environments, and a polymer material to be preferably used therein.

In consideration of such circumferences, as a result of extensive study to obtain means to sufficiently protect the living body against irritants that exist in the external environments, the inventors of the present invention have found out that the above problem could be dissolved by including a copolymer that contains a specific constituent unit at a certain amount in an external preparation for the skin and conducting the administration of the external preparation. Thus, the inventors have achieved the present invention.

In other words, the present invention relates to external preparations for the skin and copolymers, which are represented by the following (1) to (8):
(1) An external preparation for the skin, comprising a copolymer containing one or more constituent units derived from monomers represented by the general formula (I) and one or more constituent units derived from monomers represented by the general formula (II), wherein a content of the constituent units derived from the monomers represented by the general formula (I) is 5 to 25% by weight based on the total amount of all the constituent units. 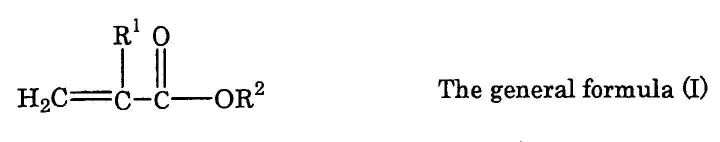 (In the formula (I), R¹ represents a hydrogen atom or an alkyl group with a carbon number of 1 to 3, and R² represents an alkyl group with a carbon number of 5 to 12 and with 8 to 21 substituted fluorine atoms.) 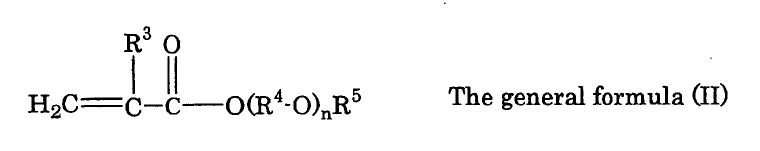 (In the formula (II), R³ represents a hydrogen atom or an alkyl group with a carbon number of 1 to 3, R⁴ represents an alkylene group with a carbon number of 2 to 4 which may have a hydroxyl group, and R⁵ represents a hydrogen atom, an aromatic group with a carbon number of 1 to 20, an aliphatic hydrocarbon group with a carbon number of 1 to 20, or an acyl group with a carbon number of 1 to 20, and n represents a numeric value of 4 to 50.)
(2) The external preparation for the skin according to the item (1), wherein the content of the constituent units derived from the monomers represented by the general formula (II) is 20 to 80% by weight based on the total amount of all the constituent units.
(3) The external preparation for the skin according to the item (1) or (2), wherein the monomers represented by the general formula (II) are represented by the following general formula (III). 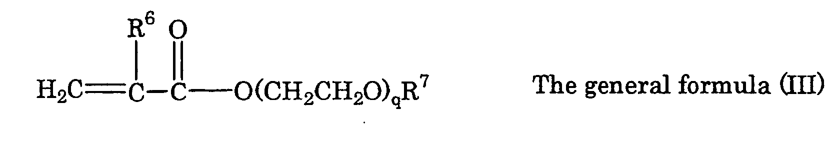 (In the formula (III), R⁶ represents a hydrogen atom or an alkyl group with a carbon number of 1 to 3, and R⁷ represents a hydrogen atom, an aromatic group with a carbon number of 1 to 20, an aliphatic hydrocarbon group with a carbon number of 1 to 20, or an acyl group with a carbon number of 1 to 20, and q represents a numeric value of 8 to 40.)
(4) The external preparation for the skin according to any one of the items (1) to (3), wherein the preparation is used for protection against irritation.
(5) A copolymer comprising:
   (1) one or more constituent units derived from monomers represented by the general formula (I) with a content of 5 to 9.8% by weight;
   (2) one or more constituent units derived from monomers represented by the general formula (II) with a content of 30.2 to 45% by weight; and
   (3) one or more constituent units derived from monomers represented by the general formula (IV) with a content of 50 to 60% by weight. 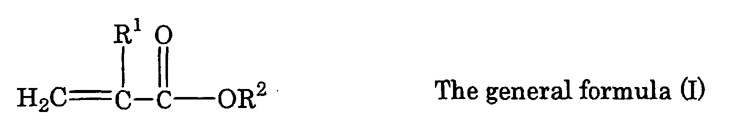 (In the formula (I), R¹ represents a hydrogen atom or an alkyl group with a carbon number of 1 to 3, and R² represents an alkyl group with a carbon number of 5 to 12 and with 8 to 21 substituted fluorine atoms.) 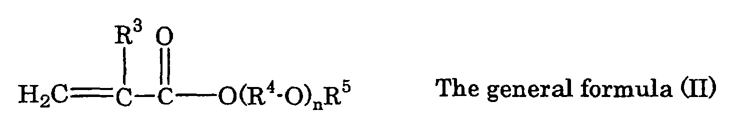 (In the formula (II), R³ represents a hydrogen atom or an alkyl group with a carbon number of 1 to 3, R⁴ represents an alkylene group with a carbon number of 2 to 4 which may have a hydroxyl group, and R⁵ represents a hydrogen atom, an aromatic group with a carbon number of 1 to 20, an aliphatic hydrocarbon group with a carbon number of 1 to 20, or an acyl group with a carbon number of 1 to 20, and n represents a numeric value of 4 to 50.) 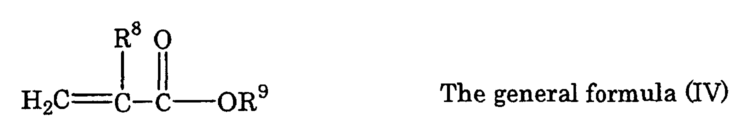 (In the formula (IV), R⁸ represents a hydrogen atom or an alkyl group with a carbon number of 1 to 3, and R⁹ represents an alkyl group with a carbon number of 1 to 3.)
(6) The copolymer according to the item (5), wherein the monomers represented by the general formula (II) are represented by the following general formula (III). 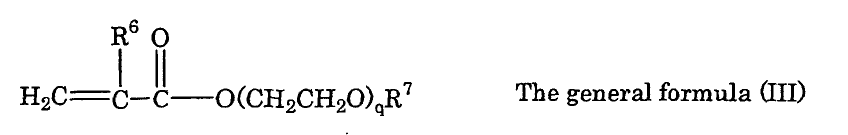 (In the formula (III), R⁶ represents a hydrogen atom or an alkyl group with a carbon number of 1 to 3, and R⁷ represents a hydrogen atom, an aromatic group with a carbon number of 1 to 20, an aliphatic hydrocarbon group with a carbon number of 1 to 20, or an acyl group with a carbon number of 1 to 20, and q represents a numeric value of 8 to 40.)
(7) An external preparation for the skin comprising the copolymer according to the item (5) or (6).
(8) The external preparation for the skin according to the item (7), wherein the preparation is used for protection against irritation.

The compounds represented by the above general formulae (I) to (IV) are known as polymer or copolymer raw materials. Furthermore, there are known technology for using copolymers, which contain constituent units derived from monomers represented by the general formula (I) and the general formula (II) (or the general formula (III)), as hydrophobic film-forming agents in cosmetics such as nail color.

However, the use of the copolymers for protecting against irritation after improving skin barrier functions by changing the composition ratio of those constituent units to make them water-soluble has not been known at all.

For the protection against irritation, it has not been known that particularly preferable among the above copolymers is a copolymer containing (1) one or more constituent units derived from monomers represented by the general formula (I) with a content of 5 to 9.8% by weight, (2) one or more constituent units derived from monomers represented by the general formula (II) with a content of 30.2 to 45% by weight, and (3) one or more constituent units derived from monomers represented by the general formula (IV) with a content of 50 to 60% by weight. Furthermore, the copolymer itself having the composition described above has not been known at all.

Hereinafter, embodiments of the present invention will be described.

### <1> External preparation for the skin of the present invention

The external preparation for the skin of the present invention contains a copolymer as an essential component, which contains one or more constituent units derived from monomers represented by the general formula (I) (hereinafter, referred to as "constituent units (I)") and one or more constituent units derived from monomers represented by the general formula (II) (hereinafter, referred to as "constituent units (II)") as its constituent units. Hereinafter, the copolymer as an essential component of the external preparation for the skin of the present invention may be referred to as "copolymer A".

### (1) Constituent unit (I)

One essential constituent unit of the copolymer A, the copolymer A being the essential component of the external preparation for the skin of the present invention, is a constituent unit (I) derived from a monomer represented by the following general formula (I).

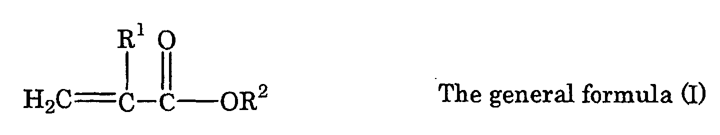

Here, in the formula (I), R¹ represents a hydrogen atom or an alkyl group with a carbon number of 1 to 3, and R² represents an alkyl group with a carbon number of 5 to 12 and with 8 to 21 substituted fluorine atoms. Examples of the alkyl group of R¹ include a methyl group, an ethyl group, a propyl group, a 1-methylethyl group, and a cyclopropyl group. Of those, R¹ is preferably a hydrogen atom or a methyl group.

Preferable examples of the alkyl group with a carbon number of 5 to 12 and with 8 to 21 substituted fluorine atoms represented by R² include: a 1H, 1H, 5H-octafluoropentyl group; a 1H,1H,2H,2H-nonafluorohexyl group; a 1H,1H,7H-dodecafluoroheptyl group; a 1H,1H-tridecafluoroheptyl group; a 1H,1H,2H,2H-heptadecafluorodecyl group; and a 1H,1H,11H-eicosafluoroundecyl group.

The compound represented by the general formula (I) as described above can be obtained not only by an esterification reaction between acid chloride derived from acrylic acid or from an α-alkyl acrylic acid and alcohol containing a fluorine atom but also by various methods. In addition, some of the compounds represented by the general formula (I) have been already commercially available, so that it is also possible to make use of them. Examples of such marketed products include the trade names "Viscoat 8F", "Viscoat 8FM", "Viscoat 17M", and "Viscoat 17FM" (each available from Osaka Organic Chemical Industry Ltd.).

In the copolymer A which is the essential component of the external preparation for the skin of the present invention, the constituent unit (I) may be of only one type. However, as far as the general formula (I) is satisfied, two or more may be combined to make up the constituent unit in total.

The copolymer A contains one or more constituent units (I) described above at a total amount of 5 to 25% by weight, preferably 5 to 20% by weight, more preferably 5 to 9.8% by weight, still more preferably 7 to 9.8% by weight with respect to all of the constituent units that constitute the copolymer.

The reason therefor is as follows. For preventing irritation, the copolymer which is the essential component needs a hydrophilic property. In this regard, it is preferable to determine the upper limit of the content of the constituent unit (I) derived from a monomer represented by the general formula (I) having a hydrophobic property within the above range. Furthermore, if the proportion of the constituent unit (I) is too small, the inhibitory effect on irritation may not be sufficiently exerted because the hydrophilic property of the copolymer is too strong.

### (2) Constituent unit (II)

Another essential constituent unit of the copolymer to be used in the external preparation for the skin of the present invention is a constituent unit (II) derived from a monomer represented by the general formula (II).

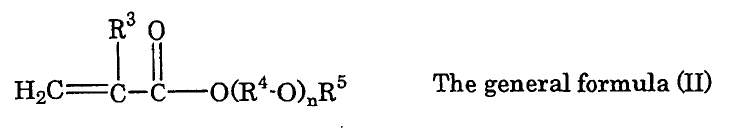

In the formula (II), R³ represents a hydrogen atom or an alkyl group with a carbon number of 1 to 3, R⁴ represents an alkylene group with a carbon number of 2 to 4 which may have a hydroxyl group, and R⁵ represents a hydrogen atom, an aromatic group with a carbon number of 1 to 20, an aliphatic hydrocarbon group with a carbon number of 1 to 20, or an acyl group with a carbon number of 1 to 20. n represents a numeric value of 4 to 50.

Examples of the alkyl group of R³ include a methyl group, an ethyl group, a propyl group, a 1-methylethyl group, and a cyclopropyl group. Preferable examples of R³ include a hydrogen atom and a methyl group. Preferable examples of the alkylene group of R⁴ include an ethylene group, a propylene group, a 1-methylethylene group, a 2-methylethylene group, a 2-hydroxypropylene group, a 1-hydroxy-2-methylethylene group, and a 2-hydroxy-1-methylethylene group. Of those, an ethylene group or a 2-hydroxypropylene group is preferable.

Of the groups of R⁵, examples of the aromatic group with a carbon number of 1 to 20 include a phenyl group, a benzyl group, a tolyl group, and a xylyl group.

Preferable examples of the aliphatic hydrocarbon group with a carbon number of 1 to 20 include a methyl group, an ethyl group, a butyl group, a tertiary butyl group, a hexyl group, a cyclohexyl group, an octyl group, a 2-ethylhexyl group, a lauryl group, a myristyl group, a palmityl group, a stearyl group, and an oleyl group. Preferable examples of the acyl group with a carbon number of 1 to 20 include an acetyl group, a butenoyl group, a capryloyl group, a caprinoyl group, a benzoyl group, a lauroyl group, a myristoyl group, a palmitoyl group, a stearoyl group, and an oleoyl group.

Of those, an alkyl group (aliphatic hydrocarbon group) with a carbon number of 1 to 12 is particularly preferable as a group of R⁵. n represents a numeric value of 4 to 50, preferably 8 to 40.

Of the monomers represented by the general formula (II), examples of particularly preferable monomers include those represented by the general formula (III). 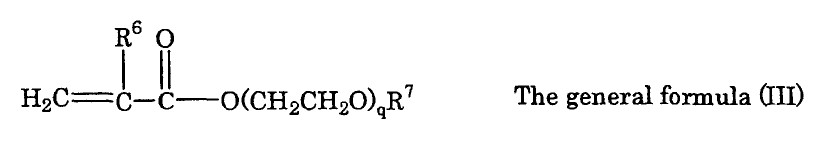 (In the formula (III), R⁶ represents a hydrogen atom or an alkyl group with a carbon number of 1 to 3, and R⁷ represents a hydrogen .atom, an aromatic group with a carbon number of 1 to 20, an aliphatic hydrocarbon group with a carbon number of 1 to 20, or an acyl group with a carbon number of 1 to 20, and q represents a numeric value of 8 to 40.)

Specific examples of the monomers represented by the general formula (III) include polyethylene glycol (9) monoacrylate, polyethylene glycol (9) monomethacrylate, methylpolyoxyethylene (9) acrylate, methylpolyoxyethylene (9) methacrylate, octylphenylpolyoxyethylene (10) acrylate, octylphenylpolyoxyethylene (10) methacrylate, nonylphenylpolyoxyethylene (15) acrylate, nonylphenylpolyoxyethylene (15) methacrylate, oleylpolyoxyethylene (18) acrylate, oleylpolyoxyethylene (18) methacrylate, polyethylene glycol (23) monoacrylate, polyethylene glycol (23) monomethacrylate, methylpolyoxyethylene (23) acrylate, methylpolyoxyethylene (23) methacrylate, cetylpolyoxyethylene (23) acrylate, cetylpolyoxyethylene (23) methacrylate, dodecylpolyoxyethylene (23) acrylate, dodecylpolyoxyethylene (23) methacrylate, lauroylpolyoxyethylene (10) acrylate, lauroylpolyoxyethylene (10) methacrylate, stearoylpolyoxyethylene (40) acrylate, and stearoylpolyoxyethylene (40) methacrylate. Note that the numbers in the brackets represent the values of n.

Those monomers can be obtained at a high yield not only by, for example, an esterification reaction between the corresponding polyethylene glycol, polyethylene glycol monoether, or polyethylene glycol monoester and acrylic or methacrylic acid chloride or an hydride but also by various methods. Many products have been already present in the market, so that the marketed products may be also utilized.

In the copolymer A which is the essential component of the external preparation for the skin of the present invention, the constituent unit (II) may be of only one type or may be a combination of two or more types as far as they satisfy the general formula (II).

The proportion of the constituent unit (II) in the copolymer is preferably 20 to 80% by weight (average weight percentage), more preferably 25 to 75% by weight, still more preferably 30.2 to 45% by weight in total with respect to all of the constituent units that constitute the copolymer.

This is, as described above, because of the necessity of both the hydrophilic property and the hydrophobic or lipophobic property with respect to the properties of the copolymer A. The constituent unit (II) derived from a monomer represented by the general formula (II) is responsible for the hydrophilic property, requiring the above content thereof for obtaining a sufficient hydrophilic property.

Furthermore, if the proportion of the constituent unit (II) is excessive, a sufficient inhibitory effect on irritation may not be exerted because of an excessively strong hydrophilic property of the copolymer.

### (3) Other constituent units

The copolymer A, which is the essential component of the external preparation for the skin of the present invention, may contain various constituent units in addition to the constituent units (I) and (II).

Of those, the copolymer A preferably contains a constituent unit derived from a monomer represented by the general formula (IV) (hereinafter, referred to as "constituent unit (IV)").

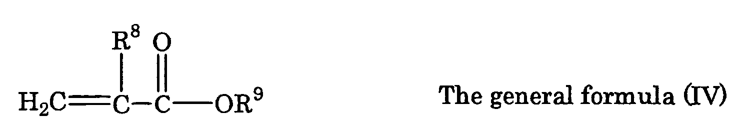

Here, in the formula (IV), R⁸ represents a hydrogen atom or an alkyl group with a carbon number of 1 to 3, and R⁹ represents an alkyl group with a carbon number of 1 to 3. Examples of the alkyl group of the groups represented by R⁸ include a methyl group, an ethyl group, a propyl group, a 1-methylethyl group, and a cyclopropyl group. Preferable examples of R⁸ include a hydrogen atom and a methyl group. Examples of the alkyl group represented by R⁹ include a methyl group, an ethyl group, a propyl group, a 1-methylethyl group, and a cyclopropyl group. Of those, a methyl group is preferable.

Specific examples of the monomers represented by the general formula (IV) include methyl acrylate, methyl methacrylate, ethyl acrylate, ethyl methacrylate, and isopropyl methacrylate, most of which can be available as marketed products.

When the copolymer A which is the essential component of the external preparation for the skin of the present invention contains as a constituent unit the constituent unit (IV), the constituent unit (IV) may be of only one type or may be a combination of two or more types as far as they satisfy the general formula (IV).

If the above copolymer contains one or more constituent units (IV), the total content of the constituent units (IV) is not specifically limited but is preferably 15 to 75% by weight, more preferably 20 to 60% by weight, still more preferably 50 to 60% by weight with respect to all of the constituent units that constitute the copolymer. The strength of a coating film to be formed by the copolymer can be increased by including the constituent units (IV) at the above rate.

The copolymer A which is the essential component of the external preparation for the skin of the present invention may optionally contain as a constituent unit a compound derived from a monomer generally used for a copolymer in addition to those constituent units so far as the effects of the present invention are not damaged. Examples of such a monomer from which an optional constituent unit is derived include: acrylic acid; methacrylic acid; maleic acid; (meth)acrylamides such as acrylamide, methacrylamide, monoalkylacrylamide, and monoalkylmethacrylamide; alkyl (meth)acrylates each having an alkyl group with a carbon number of 4 or more such as n-butyl (meth)acrylate, isobutyl (meth)acrylate, n-hexyl (meth)acrylate, and 2-ethylhexyl (meth)acrylate; aryl (meth)acrylates such as benzyl (meth)acrylate; hydroxyalkyl (meth)acrylates such as 2-hydroxyethyl (meth)acrylate, 2-hydroxypropyl (meth)acrylate, and 4-hydroxybutyl (meth)acrylate; alkoxyalkyl (meth)acrylates such as methoxymethyl (meth)acrylate and methoxyethyl (meth)acrylate; fluoroalkyl (meth)acrylates each having 7 or less substituted fluorine atoms such as 1H,1H-trifluoroethyl (meth)acrylate, 1H,1H,3H-tetrafluoroproply (meth)acrylate, and 1H-hexafluoroisopropyl (meth)acrylate; vinyl acetate; vinyl pyrrolidone; styrene; α-methylstyrene; and acrylonitrile.

### (4) Copolymer A

The copolymer A which is the essential component of the external preparation for the skin of the present invention is a copolymer that contains the above constituent units (I) and (II) and optionally the constituent unit (IV) and other constituent units in its skeleton, and the copolymer A is generally a random copolymer in which the constituent units are randomly linked together. Alternatively, however, the copolymer may be a block copolymer or a graft copolymer. In addition, the average molecular weight of the copolymer is, but not specifically limited to, on the order of preferably 5,000 to 300,000, more preferably 7,000 to 200,000 in terms of the weight average molecular weight of polyethylene glycol measured by gel permeation chromatography (GPC).

A method of producing the copolymer A is not specifically limited. However, the copolymer can be obtained by mixing monomers from which the respective constituent units are derived in a solvent and conducting a polymerization reaction according a method generally used for the polymerization of acrylic monomers.

### (5) External preparation for the skin

The external preparation for the skin of the present invention is constituted of the above copolymer A as the essential component and other optional components generally used in external preparation for the skin such as cosmetics and external medicines for the skin.

Here, the above copolymer A may contain only one type or may contain a combination of two or more types.

The content of the copolymer A in the external preparation for the skin of the present invention is preferably 0.1 to 50% by weight, more preferably 1 to 40% by weight in total. If the content of the copolymer A is too small, no inhibitory effect on irritation may be exerted. If the content is too large, preparation may become difficult to be made.

Preferable examples of the optional components except the above copolymers in the external preparation for the skin of the present invention include: hydrocarbons such as squalane, liquid paraffin, and solid paraffin; silicones such as dimethicone and phemethicone; esters such as jojoba oil and spermaceti wax; fatty acids such as stearic acid and oleic acid; higher alcohols such as behenyl alcohol, cetanol, and oleyl alcohol; triglycerides such as tallow and olive oil; nonionic surface active agents such as sorbitan sesquioleate, polyoxyethylene sorbitan monoolate, and polyoxyethylene stearate; anionic surface active agents such as sodium lauryl stearate; cationic surface active agents such as quaternary alkyl ammonium salt; polyols such as 1,3-butanediol, isoprene glycol, 1,2-pentanediol, and glycerin; powders such as crystal cellulose and crosslinking type methylpolysiloxane; thickeners such as an alkyl acrylate/methacrylate copolymer and a salt thereof, a carboxy vinylpolymer and a salt thereof, xanthan gum, and hydroxypropyl cellulose; and active ingredients such as vitamins and glycyrrhizin. Those are appropriately selected for each application.

The external preparation for the skin of the present invention can be manufactured by processing those essential and optional components according to the conventional procedure. As a method of including the copolymer in an external preparation for the skin, it is preferable that an aqueous solution of a copolymer be prepared in advance and then the solution be mixed with other components that constitute the external preparation for the skin.

The external preparation for the skin of the present invention thus obtained forms a barrier on the skin to prevent an irritant from directly contacting with the skin, so that the manifestation of skin irritation will be inhibited. The external preparation for the skin of the present invention is useful in cosmetics or external medicine for the skin.

### <2> Copolymer of the present invention

The copolymer of the present invention is particularly excellent in the inhibitory effect on skin irritation among copolymers as essential components of the external preparation for the skin of the present invention, and adopts a composition containing as the constituent units: (1) one or more constituent units (I) derived from monomers represented by the general formula (I) with a content of 5 to 9.8% by weight; (2) one or more constituent units (II) derived from monomers represented by the general formula (II) with a content of 30.2 to 45% by weight; and (3) one or more constituent units (IV) derived from monomers represented by the general formula (IV) with a content of 50 to 60% by weight.

Such a composition allows the copolymer to make a strong barrier film having the high ability of protecting against an irritant on the skin. In addition, excellent water-solubility is imparted to the copolymer, so that the copolymer will be very easily handled for preparation. The copolymer having such a composition (which may be referred to as a "copolymer B" hereinafter) is a novel compound which has never been described in the literature.

### (1) Constituent unit (I)

Of the essential constituent units which constitute the copolymer B of the present invention, the constituent unit (I) is the same as one described in the explanation for the above copolymer A. The proportion of the constituent unit (I) in the copolymer B is 5 to 9.8% by weight, preferably 6 to 9.4% by weight in total. For inhibiting irritation, the copolymer B also needs a hydrophilic property, so that the inhibitory effect on irritation may be insufficient when there is an excessive proportion of the constituent unit (I) derived from a monomer represented by the general formula (I) having a hydrophilic property. On the other hand, a small proportion thereof is not preferable because the hydrophilic property of the copolymer B is too strong in contrast and the inhibitory effect on irritation may decrease.

Now, manufacturing examples of the monomer represented by the general formula (I) from which the constituent unit (I) is derived will be described below.

### Manufacturing Example 1

1H,1H,2H,2H-nonafluoro-1-hexanol (52.8 g) as fluoro alcohol and triethylamine (50 g) were dissolved in 500 ml of tetrahydrofuran. A solution of 18.1 g of acryloyl chloride as acid chloride in 100 ml of tetrahydrofuran was dropped in this solution over 2 hours while the mixture was being cooled in ice and stirred. After the completion of the dropping, the resulting white precipitate was filtrated and tetrahydrofuran and triethylamine were removed from the filtrate using a rotary evaporator. NMR measurement revealed that the resulting compound was 1H,1H,2H,2H-nonafluorohexyl acrylate.

### Manufacturing Examples 2 to 5

Monomers represented by the general formula (I) were manufactured according to Manufacturing Example 1, except that raw materials were replaced with others. Table 1 shows the results.

**Table 1**

| | Fluoro alcohol | Acid chloride | Resulting compound |
|---|---|---|---|
| Manufacturing Example 2 | 1H,1H,7H-dodecafluoro-1-heptanol, 66.4 g | Methacryloyl chloride, 20.9 g | 1H,1H,7H-dodecafluoroheptyl methacrylate |
| Manufacturing Example 3 | 1H,1H-tridecafluoro-1-heptanol, 70.2 g | Acryloyl chloride, 18.1 g | 1H,1H-tridecafluoroheptyl acrylate |
| Manufacturing Example 4 | 1H,1H-pentadecafluoro-1-octanol, 80.0 g | Methacryloyl chloride, 20.9 g | 1H,1H-pentadecafluorooctyl methacrylate |
| Manufacturing Example 5 | 1H,1H,11H-eicosafluoro-1-undecanol, 106.4 g | Acryloyl chloride, 18.1 g | 1H,1H,11H-eicosafluoroundecyl acrylate |

### (2) Constituent unit (II)

Of the essential constituent units that constitute the copolymer B of the present invention, the constituent unit (II) is the same as one described in the explanation for the above copolymer A. In addition, of the monomers represented by the general formula (II), particularly preferable monomers are those represented by the general formula (III) described in the explanation for the above copolymer A.

The proportion of the constituent unit (II) in the copolymer B is 30.2 to 45% by weight, preferably 31 to 40% by weight in total. The properties of the copolymer B require both the hydrophilic property and the hydrophobic or lipophobic property, so that a sufficient hydrophilic property can be obtained when the proportion of the constituent unit (II) responsible for the hydrophilic property is within the above range. If the proportion of the constituent unit (II) is excessive, a sufficient inhibitory effect on irritation may not be exerted because of an excessively strong hydrophilic property of the copolymer.

Manufacturing examples of the monomer represented by the general formula (II) from which the constituent unit (II) is derived will be described below.

### Manufacturing Example 6

Polyethylene glycol #400 (80 g) as a polyethylene glycol compound and triethylamine (50 g) were dissolved in 500 ml of tetrahydrofuran. A solution of 10.5 g of methacryloyl chloride as acid chloride in 100 ml of tetrahydrofuran was dropped in this solution over 2 hours while the mixture was being cooled in ice and stirred. After the completion of the dropping, the resulting white precipitate was filtrated and tetrahydrofuran and triethylamine were removed from the filtrate using a rotary evaporator. NMR measurement revealed that the resulting compound was polyethylene glycol (9) monomethacrylate.

### Manufacturing Examples 7 to 11

Monomers represented by the general formula (II) were manufactured according to Manufacturing Example 6, except that raw materials were replaced with others. Table 2 shows the results.

**Table 2**

| | Polyethylene glycol compound | Acid chloride | Resulting compound |
|---|---|---|---|
| Manufacturing Example 7 | Polyethylene glycol (15) monononylphenyl - ether, 97.7 g | Acryloyl chloride, 9.1 g | Nonylphenylpolyoxyethylene (15) acrylate |
| Manufacturing Example 8 | Polyethylene glycol (18) monooleylether, 106 g | Methacryloyl chloride, 10.5 g | Oleylpolyoxyethylene (18) methacrylate |
| Manufacturing Example 9 | Polyethylene glycol #1000, 103g | Methacryloyl chloride, 5.2 g | Polyoxyethylene (23) monomethacrylate |
| Manufacturing Example 10 | Polyethylene glycol (10) monolaurylate, 96 g | Acryloyl chloride, 13.6 g | Lauroylpolyoxyethylene (10) acrylate |
| Manufacturing Example 11 | Polyethylene glycol (40) monostearate, 102.2 g | Acryloyl chloride, 4.5 g | Stearoylpolyoxyethylene (40) acrylate |

### (3) Constituent unit (IV)

Of the essential constituent units that constitute the copolymer B of the present invention, the constituent unit (IV) is the same as one described in the explanation for the above copolymer A. The proportion of the constituent unit (IV) in the copolymer B is 50 to 60% by weight, preferably 51 to 58% by weight in total. The strength of a coating film to be formed by the copolymer can be increased by including the constituent unit (IV) at the above rate. Thus, the film strength of the copolymer B is insufficient when the proportion of the constituent unit (IV) is too small, so that an inhibitory effect on irritation may be insufficient. On the other hand, if the proportion of the constituent unit (IV) is too large, a uniform film is hardly formed, so that a sufficient inhibitory effect on irritation may not be exerted.

Examples of the monomers represented by the general formula (IV) include methyl acrylate, methyl methacrylate, ethyl acrylate, ethyl methacrylate, and isopropyl methacrylate, most of which can be available as marketed products.

### (4) Other constituent units

The copolymer B of the present invention may optionally contain as constituent units compounds derived from monomers generally used for copolymers in addition to the above constituent units (I) to (IV) as far as the effects of the present invention are not impaired. The monomers from which the optional constituent units are derived are the same as those from which the other optional constituent units are derived described in the explanation for the above copolymer A. The contents of the optional constituent units can be suitably selected as far as the effects of the present invention are not impaired.

### (5) Copolymer B

The copolymer B of the present invention is a copolymer that contains the above constituent units (I), (II), and (IV) and, in some cases, other optional constituent units in its skeleton, and the copolymer B is generally a random copolymer in which the constituent units are randomly linked together. Alternatively, however, the copolymer may be a block copolymer or a graft copolymer. In addition, the average molecular weight of the copolymer is, but not specifically limited to, on the order of preferably 5,000 to 300,000, more preferably 7,000 to 200,000 in terms of the weight average molecular weight of polyethylene glycol measured by GPC.

A method of producing the copolymer B is not specifically limited. However, the copolymer can be obtained by mixing monomers from which the respective constituent units are derived in a solvent and conducting a polymerization reaction according to the conventional procedure.

### (6) Use

The use of the copolymer B of the present invention is not specifically limited but is particularly preferably a constituent component of an external preparation for the skin. That is, the external preparation for the skin can be constructed of the above copolymer B and other optional components generally used in external preparations for the skin such as cosmetics and external medicines for the skin.

In addition, the copolymer B of the present invention can be also used in coating compositions, surface-treating agents such as those for metals, fabrics, and glass, and so on.

Here, the above copolymer B may contain only one type or may contain a combination of two or more types.

When the copolymer B of the present invention is used for an external preparation for the skin, the content of the copolymer B in the external preparation for the skin is preferably 0.1 to 50% by weight, more preferably 1 to 40% by weight in total. If the content of the copolymer B is too small, no inhibitory effect on irritation may be exerted. If the content is too large, preparation may become difficult to be made.

In the above external preparation for the skin, the same optional components as those described in the explanation for the external preparation for the skin of the present invention which contains the above copolymer A as the essential component can be used as the optional components except the copolymer B. The external preparation for the skin of the present invention can be manufactured by processing those components according to the conventional procedure. As a method of including the copolymer B in an external preparation for the skin, it is preferable that an aqueous solution of the copolymer B be prepared in advance and then the solution be mixed with other components that constitute the external preparation for the skin.

The external preparation for the thus obtained forms a barrier on the skin to prevent an irritant from directly contacting with the skin, so that the manifestation of skin irritation will be inhibited.

### Brief Description of the Drawings

Fig. 1 is a diagram showing a ¹³C-NMR spectrum of a copolymer of the present invention obtained in Example 1.
Fig. 2 is a diagram showing a ¹H-NMR spectrum of the copolymer of the present invention obtained in Example 1.
Fig. 3 is a diagram showing an IR spectrum of the copolymer of the present invention obtained in Example 1.

### Best Mode for Carrying out the Invention

Hereinafter, the present invention will be described with reference to the examples. Needless to say, however, the present invention is not only limited to these examples.

### <Example 1>

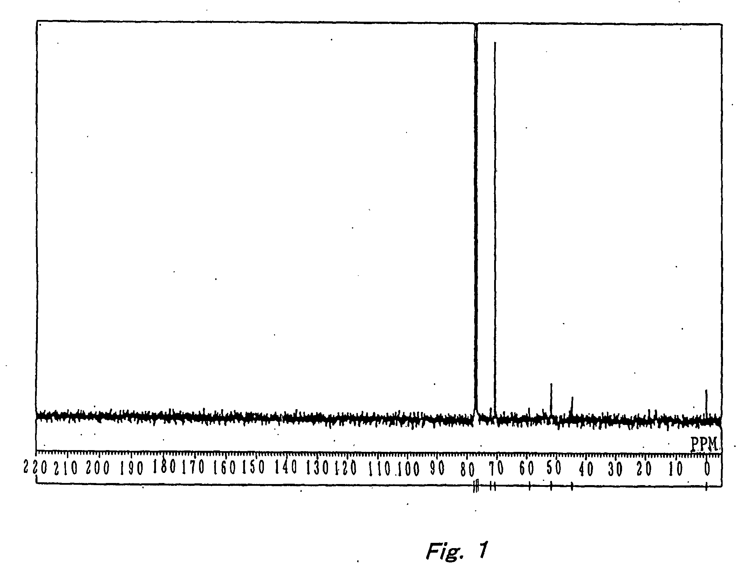

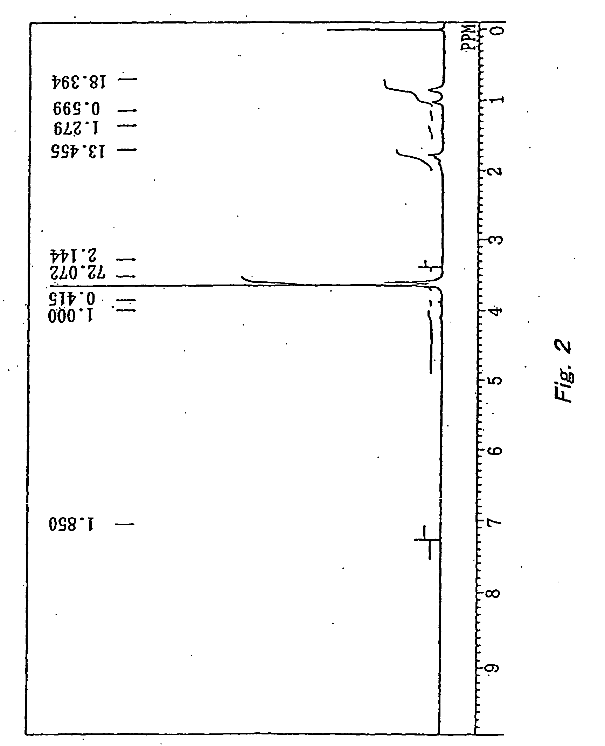

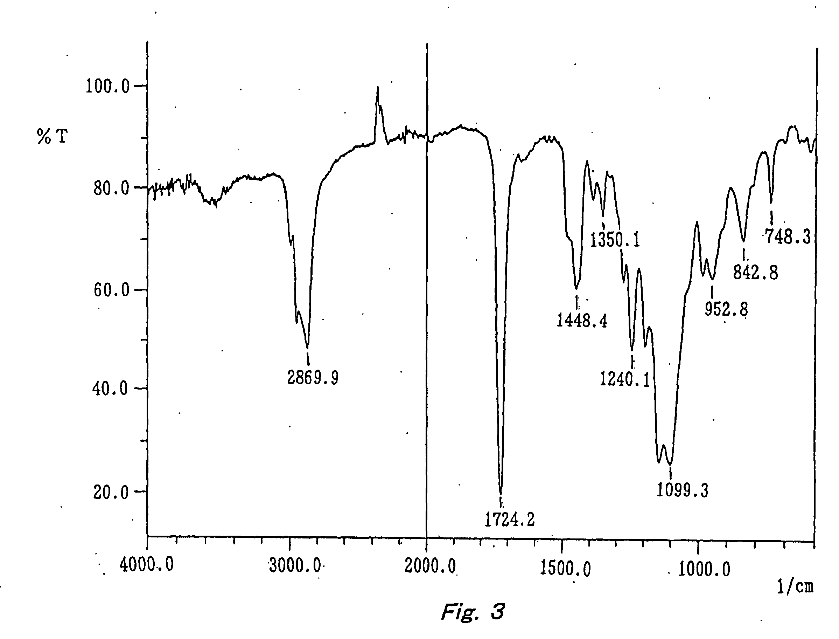

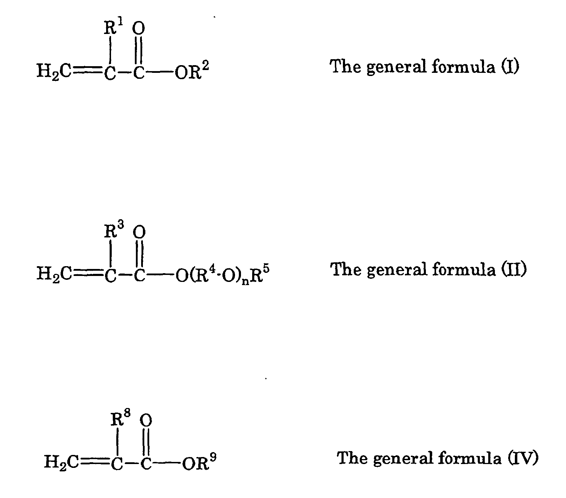

1H,1H,2H,2H-heptadecafluorodecyl acrylate (12.7 g) (available from Osaka Organic Chemical Industry Ltd., trade name: "Viscoat 17F"), methylpolyoxyethylene (23) methacrylate (52.7 g) (available from Shin-Nakamura Chemical Co., Ltd., trade name: "NK Ester M 230G"), methyl methacrylate (69.7 g) (available from Tokyo Kasei Kogyo Co., Ltd.), and a mixture solvent consisting of 300 ml of isopropyl alcohol and 200 ml of water, were collected in a flask equipped with a nitrogen-inducing tube, a cooler, and a stirring device and mixed under stirring. Nitrogen gas purge was performed for 1 hour while stirring was continued. Into thus prepared mixture, a solution of 2.3 g of ammonium peroxodisulfate in 20 ml of water was added, and furthermore the reaction was performed for 16 hours at 65°C while stirring was continued. After the completion of the reaction, isopropyl alcohol was removed by using a rotary evaporator to obtain an aqueous solution of a copolymer 1 of the present invention (molecular weight = 45,000; The molecular weight mentioned herein means a weight average molecular weight in terms of polyethylene glycol measured by GPC, the same shall apply hereinafter). The results of ¹³C-NMR, ¹H-NMR, and IR measurements conducted on the copolymer are shown in Figs. 1 to 3, respectively. Those measurements show that the copolymer of the present invention is obtained.

### <Example 2>

1H,1H,7H-dodecafluoroheptyl methacrylate (1.2 g) (the compound of Manufacturing Example 2), 1H,1H,11H-eicosafluoroundecyl acrylate (3.0 g) (the compound of Manufacturing Example 5), stearoylpolyoxyethylene (40) acrylate (22.8 g) (Manufacturing Example 11), ethyl acrylate (33.0 g), and a mixture solvent consisting of 200 ml of ethyl alcohol and 100 ml of water, were collected in a flask equipped with a nitrogen-inducing tube, a cooler, and a stirring device and mixed under stirring. Nitrogen gas purge was performed for 1 hour while stirring was continued. Into thus prepared mixture, a solution of 1.0 g of ammonium peroxodisulfate dissolved in 10 ml of water was added, and furthermore the reaction was performed for 12 hours at 70°C while stirring was continued. After the completion of the reaction, isopropyl alcohol was removed by using a rotary evaporator to obtain an aqueous solution of a copolymer 2 of the present invention (molecular weight = 150,000).

### <Example 3>

1H,1H-tridecafluoroheptyl acrylate (3.1 g) (the compound of Manufacturing Example 3), methylpolyoxyethylene (9) acrylate (9.5 g) (available from Shin-Nakamura Chemical Co., Ltd., trade name: "NK Ester AM 90G"), oleylpolyoxyethylene (18) methacrylate (12.0 g) (the compound of Manufacturing Example 8), isopropyl methacrylate (35.4 g) (available from Tokyo Kasei Kogyo Co., Ltd.), and 300 ml of ethyl acetate were collected in a flask equipped with a nitrogen-inducing tube, a cooler, and a stirring device and mixed under stirring. Nitrogen gas purge was performed for 1 hour while stirring was continued. A solution of 0.5 g of benzoyl peroxide in 10 ml of ethyl acetate was added to the mixture, and furthermore the reaction was performed by 8 hours of reflux while stirring was continued. After the completion of the reaction, water was added to the solution, followed by flashing to obtain an aqueous solution of a copolymer 3 of the present invention (molecular weight = 10,000).

### <Example 4>

1H,1H,11H-eicosafluoroundecyl acrylate (4.8 g) (the compound of Manufacturing Example 5), stearoylpolyoxyethylene (40) acrylate (22.2 g) (the compound of Manufacturing Example 11), methyl methacrylate (24.0 g) (available from Tokyo Kasei Kogyo Co., Ltd.), ethyl methacrylate (9.0 g) (available from Tokyo Kasei Kogyo Co., Ltd.), and a mixture solvent consisting of 200 ml of isopropyl alcohol and 100 ml of water, were collected in a flask equipped with a nitrogen-inducing tube, a cooler, and a stirring device and mixed under stirring. Nitrogen gas purge was performed for 1 hour while stirring was continued. Into thus prepared mixture, a solution of 1.0 g of ammonium peroxodisulfate in 10 ml of water was added, and furthermore the reaction was performed for 18 hours at 70°C while stirring was continued. After the completion of the reaction, isopropyl alcohol was removed by using a rotary evaporator to obtain an aqueous solution of a copolymer 4 of the present invention (molecular weight = 70,000) .

### <Test Example 1>

The copolymers 1 to 4 of Examples 1 to 4 described above (the copolymer B of the present invention), copolymers the manufacturing examples of which are described below, which do not belong to the copolymer B of the present invention but to the copolymer A which is the essential component of the skin external composition of the present invention (Manufacturing Examples 12 to 17), and a copolymer (Manufacturing Example 18) which does not belong to the copolymer A were investigated for barrier function on the skin. The barrier function was evaluated by the impermeability of the substance to the skin.

That is, a sample-coating area was formed on the skin and the color thereof was measured. A 5% aqueous solution of each of the copolymers of Manufacturing Examples 12 to 18 and Examples 1 to 4 was applied on this area. After the solution was let stand for 5 minutes, glyceryl triethylhexanoate containing 0.05% Red No. 223 (D&C Red No.21) (R223/TIO) or water containing 0.05% Red No. 213 (D&C Red No.19) (R213/water), where each of them is a skin-stainable dye, was applied on the coating film of the polymer and left alone for 30 minutes. The coating film was removed with wet tissue, and then color measurement (visual observation) was performed after a wait for the disappearance of the redness of the skin caused by physical irritation. The penetrating powers of oil and water were evaluated in terms of variations in redness, namely value a, before and after the skin-stainability experiment.

Furthermore, such an effect was evaluated for persistence by the following method. At first, a 5% aqueous solution of each copolymer was applied and left alone for 5 minutes, and then the running water was poured onto the applied area, lightly rubbed for 5 minutes, and lightly wiped off with tissue, followed by applying a skin-stainable dye solution on the coating film of the copolymer and leaving it alone for 30 minutes. The coating film was removed with wet tissue, and then color measurement (visual observation) was performed after a wait for the disappearance of the redness of the skin caused by physical irritation. Likewise, the penetrating powers were evaluated in terms of variations in redness, namely value a, before and after the skin-stainability experiment.

The results thereof are shown in Table 3. Those results show that the copolymer A which is the essential component of the external preparation for the skin of the present invention has an excellent effect of blocking the substance. Most of all, in the copolymer B of the present invention, it turns out that this effect is remarkable.

### Manufacturing Example 12

1H,lH-pentadecafluorooctyl methacrylate (8.0 g) (the compound of Manufacturing Example 4), lauroylpolyoxyethylene (10) acrylate (32.0 g) (the compound of Manufacturing Example 10), and 200 ml of isopropyl alcohol were collected in a flask equipped with a nitrogen-inducing tube, a cooler, and a stirring device and mixed under stirring. Nitrogen gas purge was performed for 1 hour while stirring was continued. A solution of 0.5 g of benzoyl peroxide in 10 ml of isopropyl alcohol was added to the mixture, and furthermore the reaction was performed for 8 hours at 80°C while stirring was continued. After the completion of the reaction, a large amount of n-hexane was added to the solution, and the resulting precipitate was dried and then dissolved in water to obtain an aqueous solution of a copolymer 5 for use in the present invention (molecular weight = 8,000).

### Manufacturing Example 13

1H,1H-tridecafluoroheptyl acrylate (9.0 g) (the compound of Manufacturing Example 3), polyethylene glycol (9) monomethacrylate (36.0 g) (the compound of Manufacturing Example 6), n-butyl methacrylate (15.0 g) (available from Tokyo Kasei Kogyo Co., Ltd.), and 300 ml of ethyl acetate were collected in a flask equipped with a nitrogen-inducing tube, a cooler, and a stirring device and mixed under stirring. Nitrogen gas purge was performed for 1 hour while stirring was continued. A solution of 0.5 g of benzoyl peroxide in 10 ml of ethyl acetate was added to the mixture, and furthermore the reaction was performed by 8 hours of reflux while stirring was continued. After the completion of the reaction, water was added to the solution, followed by flashing to obtain an aqueous solution of a copolymer 6 for use in the present invention (molecular weight = 15,000) .

### Manufacturing Example 14

1H,1H,2H,2H-heptadecafluorodecyl acrylate (6.0 g) (available from Osaka Organic Chemical Industry Ltd., trade name: "Viscoat 17F"), 1H,1H-tridecafluoroethyl methacrylate (18.0 g) (available from Osaka Organic Chemical Industry Ltd., trade name: "Viscoat 3FM"), methylpolyoxyethylene (23) methacrylate (24.0 g) (available from Shin-Nakamura Chemical Co., Ltd., trade name: "NK Ester M230G"), 2-hydroxyethyl methacrylate (12.0 g) (available from Tokyo Kasei Kogyo Co., Ltd.), and a mixture solvent consisting of 180 ml of isopropyl alcohol and 120 ml of water, were collected in a flask equipped with a nitrogen-inducing tube, a cooler, and a stirring device and mixed under stirring. Nitrogen gas purge was performed for 1 hour while stirring was continued. Into thus prepared mixture, a solution of 1.0 g of ammonium peroxodisulfate in 10 ml of water was added, and furthermore the reaction was performed for 12 hours at 70°C while stirring was continued. After the completion of the reaction, isopropyl alcohol was removed by using a rotary evaporator to obtain an aqueous solution of a copolymer 7 for use in the present invention (molecular weight = 28,000).

### Manufacturing Example 15

1H,1H,11H-eicosafluoroundecyl acrylate (1.2 g) (the compound of Manufacturing Example 5), 1H,1H,5H-octafluoropentyl methacrylate (1.6 g) (available from Osaka Organic Chemical Industry Ltd., trade name: "Viscoat 8FM"), oleylpolyoxyethylene (18) methacrylate (26.0 g) (the compound of Manufacturing Example 8), styrene (11.2 g) (available from Tokyo Kasei Kogyo Co., Ltd.), and 250 ml of toluene were collected in a flask equipped with a nitrogen-inducing tube, a cooler, and a stirring device and mixed under stirring. Nitrogen gas purge was performed for 1 hour while stirring was continued. A solution of 0.5 g of azobisisobutyronitrile in 10 ml of toluene were added to the mixture, and furthermore the reaction was performed for 8 hours at 70°C while stirring was continued. After the completion of the reaction, water was added to the solution, followed by flashing to obtain an aqueous solution of a copolymer 8 for use in the present invention (molecular weight = 20,000) .

### Manufacturing Example 16

1H,1H,2H,2H-nonafluorohexyl acrylate (3.6 g) (the compound of Manufacturing Example 1), nonylphenylpolyoxyethylene (15) acrylate (18.0 g) (the compound of Manufacturing Example 7), polyethylene glycol (23) monomethacrylate (18.0 g) (the compound of Manufacturing Example 9), ethyl acrylate (20.4 g) (available from Tokyo Kasei Kogyo Co., Ltd.), and a mixture solvent consisting of 150 ml of ethyl alcohol and 150 ml of water, were collected in a flask equipped with a nitrogen-inducing tube, a cooler, and a stirring device and mixed under stirring. Nitrogen gas purge was performed for 1 hour while stirring was continued. Into thus prepared mixture, a solution of 1.0 g of potassium peroxodisulfate in 10 ml of water was added, and furthermore the reaction was performed for 12 hours at 80°C while stirring was continued. After the completion of the reaction, ethyl alcohol was removed by using a rotary evaporator to obtain an aqueous solution of a copolymer 9 for use in the present invention (molecular weight = 100,000).

### Manufacturing Example 17

1H,1H,2H,2H-heptadecafluorodecyl acrylate (5.6 g) (available from Osaka Organic Chemical Industry Ltd., trade name: "Viscoat 17F"), methylpolyoxyethylene (23) methacrylate (24.4 g) (available from Shin-Nakamura Chemical Co., Ltd., trade name: "NK Ester M 230G", methyl methacrylate (18.0 g) (available from Tokyo Kasei Kogyo Co., Ltd.), 2-hydroxyethyl methacrylate (12.0 g) (available from Tokyo Kasei Kogyo Co., Ltd.), and a mixture solvent consisting of 180 ml of isopropyl alcohol and 120 ml of water, were collected in a flask equipped with a nitrogen-inducing tube, a cooler, and a stirring device and mixed under stirring. Nitrogen gas purge was performed for 1 hour while stirring was continued. Into thus prepared mixture, a solution of 1.0 g of ammonium peroxodisulfate in 10 ml of water was added, and furthermore the reaction was performed for 12 hours at 70°C while stirring was continued. After the completion of the reaction, isopropyl alcohol was removed by using a rotary evaporator to obtain an aqueous solution of a copolymer 10 for use in the present invention (molecular weight = 32,000).

### Manufacturing Example 18 (Comparative Example; Copolymer not belonging to Copolymer A)

1H,1H,2H,2H-heptadecafluorodecyl acrylate (2.4 g) (available from Osaka Organic Chemical Industry Ltd., trade name: "Viscoat 17F"), methylpolyoxyethylene (23) methacrylate (36.0 g) (available from Shin-Nakamura Chemical Co., Ltd., trade name: "NK Ester M 230G"), 2-hydroxyethyl methacrylate (21.6 g) (available from Tokyo Kasei Kogyo Co., Ltd.), and a mixture solvent consisting of 180ml of isopropyl alcohol and 120 ml of water, were collected in a flask equipped with a nitrogen-inducing tube, a cooler, and a stirring device and mixed under stirring. Nitrogen gas purge was performed for 1 hour while stirring was continued. A solution of 1.0 g of ammonium peroxodisulfate in 10 ml of water was added and furthermore the reaction was performed for 12 hours at 70°C while stirring was continued. After the completion of the reaction, isopropyl alcohol was removed by using a rotary evaporator to obtain a target aqueous solution of a copolymer 11.

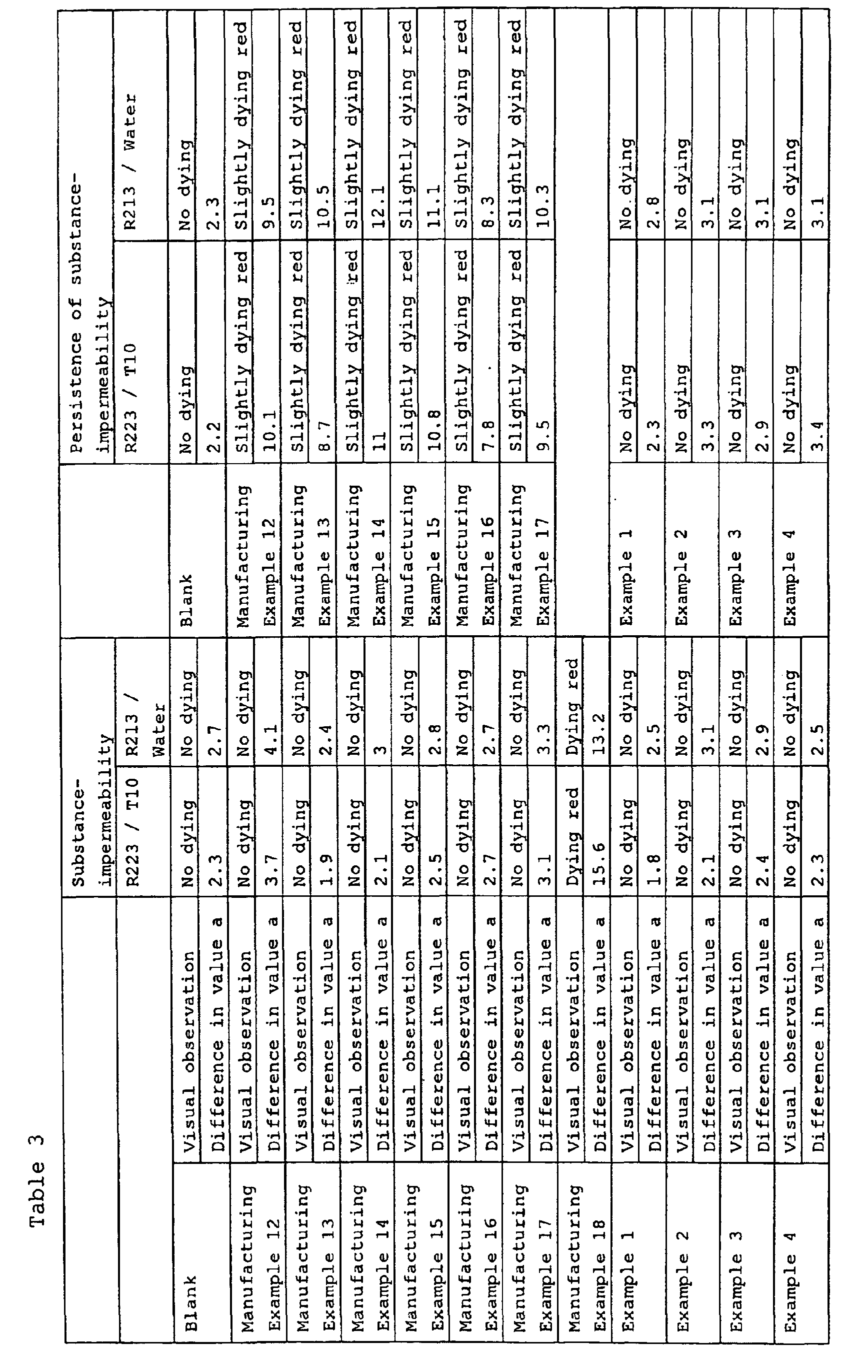

### <Examples 5 to 8 and Comparative Example 1>

A makeup base cream as the composition (the external preparation for the skin) of the present invention was prepared according to the following formulation. That is, the cream was obtained by respectively heating (a) a mixture of Components 1 to 8 of Table 4 and (b) a mixture of Components 9 to 20 of Table 4 up to 80°C, gradually adding (b) to (a), and emulsifying the resultant, and cooling with stirring. For representing the formulation of the external preparation for the skin in the tables below, each of numerical values in the tables is expressed in % by weight.

**Table 4**

| No. | Component name | Example | | | | Comparative Example |
|---|---|---|---|---|---|---|
| | | 5 | 6 | 7 | 8 | 1 |
| 1 | Cyclodimethicone | 4 | 4 | 4 | 4 | 4 |
| 2 | Cetyl octanoate | 3 | 3 | 3 | 3 | 3 |
| 3 | Cetanol | 1 | 1 | 1 | 1 | 1 |
| 4 | Squalane | 1 | 1 | 1 | 1 | 1 |
| 5 | Stearic acid | 1 | 1 | 1 | 1 | 1 |
| 6 | Talc | 2 | 2 | 2 | 2 | 2 |
| 7 | Sorbitan distearate | 0.8 | 0.8 | 0.8 | 0.8 | 0.8 |
| 8 | Glyceryl stearate | 1 | 1 | 1 | 1 | 1 |
| 9 | Alkyl acrylate copolymer emulsion | 0.6 | 0.6 | 0.6 | 0.6 | 0.6 |
| 10 | Xanthan gum | 0.2 | 0.2 | 0.2 | 0.2 | 0.2 |
| 11 | Copolymer 5 | 10 | | | | |
| 12 | Copolymer 9 | | 10 | | | |
| 13 | Copolymer 7 | | | 10 | | |
| 14 | Copolymer 1 | | | | 10 | |
| 15 | Copolymer 11 | | | | | 10 |
| 16 | Glycerin | 6 | 6 | 6 | 6 | 6 |
| 17 | 1,3-butanediol | 2 | 2 | 2 | 2 | 2 |
| 18 | Purified water | 66.7 | 66.7 | 66.7 | 66.7 | 66.7 |
| 19 | Triethanolamine | 0.3 | 0.3 | 0.3 | 0.3 | 0.3 |
| 20 | Antiseptic agent | 0.4 | 0.4 | 0.4 | 0.4 | 0.4 |

### <Examples 9 to 12 and Comparative Example 2>

A foundation as the composition of the present invention was prepared according to the following formulation. That is, the foundation was obtained by respectively heating (a) a mixture of Components 1 to 3 of Table 5, (b) Component 4 of Table 5, (c) a mixture of Components 5 to 9 of Table 5, and (d) a mixture of Components 10 o 14 of Table 5 up to 80°C, well kneading (a) and (b), diluting the resultant with (c), dispersing (e) a mixture of Components 15 to 18 of Table 5, gradually adding (d) thereto, emulsifying the resultant, and cooling with stirring.

**Table 5**

| No. | Component name | Example | | | | Comparative Example |
|---|---|---|---|---|---|---|
| | | 9 | 10 | 11 | 12 | 2 |
| 1 | 70% maltitol aqueous solution | 5 | 5 | 5 | 5 | 5 |
| 2 | Glycerin | 2 | 2 | 2 | 2 | 2 |
| 3 | 1,3-butanediol | 5 | 5 | 5 | 5 | 5 |
| 4 | Triglyceryl diisostearate | 5 | 5 | 5 | 5 | 5 |
| 5 | Liquid paraffin | 15 | 15 | 15 | 15 | 15 |
| 6 | Carnauba wax | 2 | 2 | 2 | 2 | 2 |
| 7 | Microcrystalline Wax | 3 | 3 | 3 | 3 | 3 |
| 8 | p-methoxy isooctyl cinnamate | 2 | 2 | 2 | 2 | 2 |
| 9 | Perfluoroether | | | | | 10 |
| 10 | Purified water | 37 | 37 | 37 | 37 | 37 |
| 11 | Copolymer 6 | 10 | | | | |
| 12 | Copolymer 8 | | 10 | | | |
| 13 | Copolymer 10 | | | 10 | | |
| 14 | Copolymer 2 | | | | 10 | |
| 15 | Titanium oxide | 9 | 9 | 9 | 9 | 9 |
| 16 | Talc | 3 | 3 | 3 | 3 | 3 |
| 17 | Red iron oxide | 1 | 1 | 1 | 1 | 1 |
| 18 | Yellow iron oxide | 1 | 1 | 1 | 1 | 1 |

### <Examples 13 to 16 and Comparative Example 3>

An aqueous gel as the composition of the present invention was prepared according to the following formulation. That is, the aqueous gel was obtained by respectively mixing (a) a mixture of Components 1 to 10 of Table 6 and (b) a mixture of Components 11 and 12 of Table 6 under stirring to prepare uniform solutions. While (a) was stirred, (b) was gradually added, after which the resultant was left standing.

**Table 6**

| No. | Component name | Example | | | | Comparative Example |
|---|---|---|---|---|---|---|
| | | 13 | 14 | 15 | 16 | 3 |
| 1 | Carbopol | 0.5 | 0.5 | 0.5 | 0.5 | 0.5 |
| 2 | 1,3-butanediol | 10 | 10 | 10 | 10 | 10 |
| 3 | Ethanol | 5 | 5 | 5 | 5 | 5 |
| 4 | Copolymer 5 | 15 | | | | |
| 5 | Copolymer 8 | | 15 | | | |
| 6 | Copolymer 10 | | | 15 | | |
| 7 | Copolymer 3 | | | | 15 | |
| 8 | Copolymer 11 | | | | | 15 |
| 9 | Purified water | 53.9 | 53.9 | 53.9 | 53.9 | 53.9 |
| 10 | Antiseptic agent | 0.4 | 0.4 | 0.4 | 0.4 | 0.4 |
| 11 | Potassium hydroxide | 0.2 | 0.2 | 0.2 | 0.2 | 0.2 |
| 12 | Purified water | 15 | 15 | 15 | 15 | 15 |

### <Examples 17 to 19 and Comparative Example 4>

A spray as the composition of the present invention was prepared according to the following formulation shown in Table 7 below. That is, a spray solution was obtained by sufficiently mixing Components 1 to 8 with stirring.

**Table 7**

| No. | Component name | Example | | | Comparative Example |
|---|---|---|---|---|---|
| | | 17 | 18 | 19 | 4 |
| 1 | Polyethylene glycol #6000 | 5 | 5 | 5 | 5 |
| 2 | Ethanol | 5 | 5 | 5 | 5 |
| 3 | Purified water | 69.8 | 69.8 | 69.8 | 69.8 |
| 4 | Antiseptic agent | 0.2 | 0.2 | 0.2 | 0.2 |
| 5 | Copolymer 8 | 20 | | | |
| 6 | Copolymer 10 | | 20 | | |
| 7 | Copolymer 4 | | | 20 | |
| 8 | Copolymer 11 | | | | 20 |

### <Examples 20 to 22 and Comparative Example 5>

A lotion as the composition of the present invention was prepared according to the following formulation shown in Table 8 below. That is, the lotion was obtained by sufficiently mixing Components 1 to 8 with stirring.

**Table 8**

| No. | Component name | Example | | | Comparative Example |
|---|---|---|---|---|---|
| | | 20 | 21 | 22 | 5 |
| 1 | Glycerin | 3 | 3 | 3 | 3 |
| 2 | 1,2-pentanediol | 5 | 5 | 5 | 5 |
| 3 | Ethanol | 5 | 5 | 5 | 5 |
| 4 | Copolymer 5 | 5 | | | |
| 5 | Copolymer 6 | | 5 | | |
| 6 | Copolymer 2 | | | 5 | |
| 7 | Copolymer 11 | | | | 5 |
| 8 | Purified water | 82 | 82 | 82 | 82 |

### <Test Example 2: Evaluation of inhibitory effect on feeling of irritation>

The compositions (external preparation for the skin) of Examples 5 to 22 and Comparative Examples 1 to 5 were applied on the cheek areas under the eyes, respectively.

After the compositions were left alone for 5 minutes, non woven fabric dampened with an irritant solution or an irritant-containing emulsion was placed on the composition-applied area. The feeling of irritation was scored in every minute by the following five stages, and such evaluation was repeated until 5 minutes elapsed. The total of scores of 5-minute duration was defined as an irritation score. The same evaluation was conducted on each of 20 panelists and the total of the irritation scores was defined as an irritation value of each irritant. The results are shown in Table 9. Numerical values shown in the table are irritation values.

### Irritation Scores

- 0:: Feeling nothing at all
- 1:: Feeling something wrong
- 2:: Feeling irritation a little
- 3:: Feeling irritation distinctly
- 4:: Feeling irritation considerably
- 5:: Intolerable

**Table 9**

| | Irritant-containing emulsion | | | Irritant aqueous solution | | |
|---|---|---|---|---|---|---|
| | Butyl parabene 0.2% | Methyl salicylate 0.5% | Capsaicin 0.05% | Methyl parabene 0.5% | Lactic acid 0.2% | Citric acid 0.2% |
| Blank | 310 | 302 | 323 | 301 | 325 | 312 |
| Example 5 | 91 | 78 | 90 | 95 | 81 | 92 |
| Example 6 | 83 | 90 | 100 | 87 | 83 | 101 |
| Example 7 | 95 | 93 | 90 | 92 | 95 | 89 |
| Example 9 | 89 | 103 | 96 | 88 | 87 | 92 |
| Example 10 | 81 | 92 | 85 | 97 | 91 | 102 |
| Example 11 | 84 | 86 | 83 | 95 | 94 | 87 |
| Example 13 | 82 | 94 | 79 | 84 | 92 | 95 |
| Example 14 | 96 | 85 | 87 | 91 | 100 | 84 |
| Example 15 | 87 | 87 | 91 | 96 | 88 | 93 |
| Example 17 | 90 | 89 | 92 | 93 | 96 | 86 |
| Example 18 | 93 | 81 | 83 | 95 | 83 | 90 |
| Example 20 | 78 | 80 | 94 | 85 | 83 | 85 |
| Example 21 | 81 | 92 | 100 | 97 | 85 | 82 |
| Example 8 | 84 | 83 | 83 | 95 | 88 | 92 |
| Example 12 | 85 | 82 | 86 | 85 | 81 | 83 |
| Example 16 | 89 | 81 | 83 | 84 | 83 | 82 |
| Example 19 | 93 | 79 | 94 | 85 | 91 | 92 |
| Example 22 | 82 | 85 | 82 | 85 | 81 | 83 |
| Comparative Example 1 | 301 | 295 | 288 | 299 | 305 | 301 |
| Comparative Example 2 | 288 | 305 | 297 | 278 | 283 | 290 |
| Comparative Example 3 | 295 | 303 | 306 | 287 | 291 | 289 |
| Comparative Example 4 | 282 | 325 | 315 | 286 | 309 | 288 |
| Comparative Example 5 | 299 | 315 | 314 | 295 | 287 | 297 |

### <Test Example 3: Evaluation of persistence for inhibitory effect on feeling of irritation>

The compositions of Examples 5 to 22 were applied on the cheek areas under the eyes, respectively. After the compositions were left alone for 5 minutes, the composition-applied area was dabbed with wet tissue. This operation was repeated 20 times. After that, non woven fabric dampened with an irritant aqueous solution or an irritant-containing emulsion was placed on the composition-applied area. The feeling of irritation was scored in every minute by the above-mentioned same five stages, and such an evaluation was repeated until 5 minutes elapsed. The total of scores of 5-minute duration was referred to as an irritation score. The same evaluation was conducted on each of 20 panelists and the total of the irritation scores was referred to as an irritation value of each irritant. The results are shown in Table 10.

**Table 10**

| | Irritant-containing emulsion | | | Irritant aqueous solution | | |
|---|---|---|---|---|---|---|
| | Butyl parabene 0.2% | Methyl salicylate 0.5% | Capsaicin 0.05% | Methyl parabene 0.5% | Lactic acid 0.2% | Citric acid 0.2% |
| Blank | 308 | 310 | 314 | 305 | 314 | 317 |
| Example 5 | 203 | 208 | 231 | 215 | 222 | 200 |
| Example 6 | 215 | 209 | 212 | 201 | 219 | 209 |
| Example 7 | 204 | 206 | 221 | 217 | 209 | 215 |
| Example 9 | 216 | 213 | 215 | 221 | 222 | 209 |
| Example 10 | 203 | 224 | 213 | 215 | 200 | 204 |
| Example 11 | 241 | 223 | 241 | 232 | 208 | 215 |
| Example 13 | 212 | 221 | 216 | 223 | 212 | 218 |
| Example 14 | 207 | 217 | 229 | 214 | 213 | 202 |
| Example 15 | 215 | 212 | 227 | 208 | 213 | 223 |
| Example 17 | 208 | 221 | 229 | 217 | 216 | 204 |
| Example 18 | 210 | 220 | 211 | 210 | 220 | 200 |
| Example 20 | 225 | 209 | 214 | 206 | 213 | 211 |
| Example 21 | 205 | 207 | 218 | 204 | 203 | 232 |
| Example 8 | 96 | 84 | 99 | 98 | 93 | 94 |
| Example 12 | 102 | 92 | 89 | 105 | 88 | 101 |
| Example 16 | 100 | 95 | 90 | 98 | 93 | 100 |
| Example 19 | 98 | 89 | 97 | 92 | 98 | 102 |
| Example 22 | 100 | 95 | 99 | 97 | 98 | 97 |

In this manner, it is evident from Test Example 2 that any of the external preparation for the skin of the present invention has an excellent inhibitory action on irritation. In addition, it is also evident from Test Example 3 that such an irritation-inhibitory effect is sustained for a significantly long period of time when the copolymer is the copolymer B of the present invention (Examples 8, 12, 16, 19, and 22).

### Industrial Applicability

According to the present invention, means for sufficiently protecting the living body against irritants existing in the external environments can be provided.

## Claims

1. An external preparation for the skin, comprising a copolymer containing one or more constituent units derived from monomers represented by the general formula (I) and one or more constituent units derived from monomers represented by the general formula (II), wherein a content of the constituent units derived from the monomers represented by the general formula (I) is 5 to 25% by weight based on a total amount of all the constituent units. 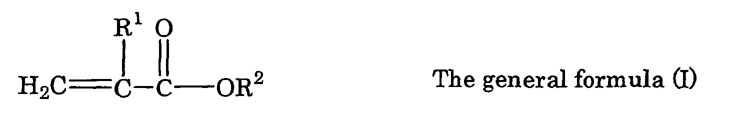 (In the formula (I), R¹ represents a hydrogen atom or an alkyl group with a carbon number of 1 to 3, and R² represents an alkyl group with a carbon number of 5 to 12 and with 8 to 21 substituted fluorine atoms.) 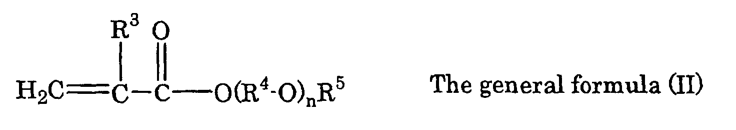 (In the formula (II), R³ represents a hydrogen atom or an alkyl group with a carbon number of 1 to 3, R⁴ represents an alkylene group with a carbon number of 2 to 4 which may have a hydroxyl group, and R⁵ represents a hydrogen atom, an aromatic group with a carbon number of 1 to 20, an aliphatic hydrocarbon group with a carbon number of 1 to 20, or an acyl group with a carbon number of 1 to 20, and n represents a numeric value of 4 to 50.)

2. The external preparation for the skin according to claim 1, wherein the content of the constituent units derived from the monomers represented by the general formula (II) is 20 to 80% by weight based on the total amount of all the constituent units.

3. The external preparation for the skin according to claim 1 or 2, wherein the monomers represented by the general formula (II) are represented by the following general formula (III). 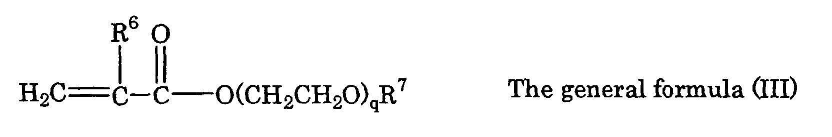 (In the formula (III), R⁶ represents a hydrogen atom or an alkyl group with a carbon number of 1 to 3, and R⁷ represents a hydrogen atom, an aromatic group with a carbon number of 1 to 20, an aliphatic hydrocarbon group with a carbon number of 1 to 20, or an acyl group with a carbon number of 1 to 20, and q represents a numeric value of 8 to 40.)

4. The external preparation for the skin according to any one of claims 1 to 3, wherein the preparation is used for protection against irritation.

5. A copolymer comprising:
(1) one or more constituent units derived from monomers represented by the general formula (I) with a content of 5 to 9.8% by weight;
(2) one or more constituent units derived from monomers represented by the general formula (II) with a content of 30.2 to 45% by weight; and
(3) one or more constituent units derived from monomers represented by the general formula (IV) with a content of 50 to 60% by weight. 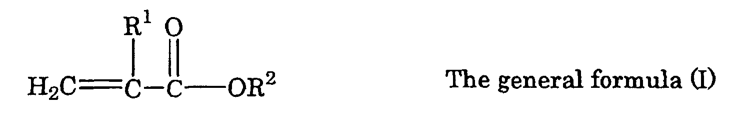 (In the formula (I), R¹ represents a hydrogen atom or an alkyl group with a carbon number of 1 to 3, and R² represents an alkyl group with a carbon number of 5 to 12 and with 8 to 21 substituted fluorine atoms.) 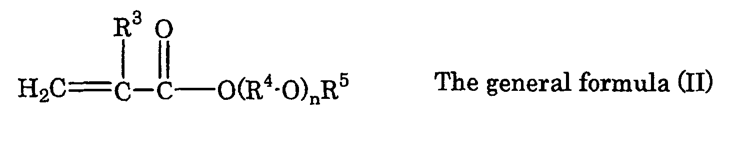 (In the formula (II), R³ represents a hydrogen atom or an alkyl group with a carbon number of 1 to 3, R⁴ represents an alkylene group with a carbon number of 2 to 4 which may have a hydroxyl group, and R⁵ represents a hydrogen atom, an aromatic group with a carbon number of 1 to 20, an aliphatic hydrocarbon group with a carbon number of 1 to 20, or an acyl group with a carbon number of 1 to 20, and n represents a numeric value of 4 to 50.) 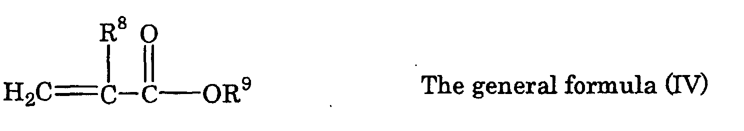 (In the formula (IV), R⁸ represents a hydrogen atom or an alkyl group with a carbon number of 1 to 3, and R⁹ represents an alkyl group with a carbon number of 1 to 3.)

6. The copolymer according to claim 5, wherein the monomers represented by the general formula (II) are represented by the following general formula (III). 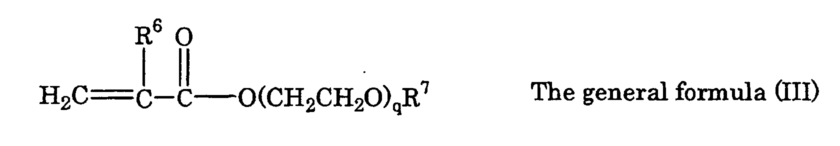 (In the formula (III), R⁶ represents a hydrogen atom or an alkyl group with a carbon number of 1 to 3, and R⁷ represents a hydrogen atom, an aromatic group with a carbon number of 1 to 20, an aliphatic hydrocarbon group with a carbon number of 1 to 20, or an acyl group with a carbon number of 1 to 20, and q represents a numeric value of 8 to 40.)

7. An external preparation for the skin comprising the copolymer according to claim 5 or 6.

8. The external preparation for the skin according to claim 7, wherein the preparation is used for protection against irritation.
